# EUROPEAN PATENT APPLICATION

(11) **EP 4 140 302 A1**
(43) Date of publication of application: **01.03.2023**
(21) Application number: 21792498.4
(22) Date of filing: 20.04.2021
(51) Int. Cl.: A01N 37/06, A01N 25/18, C07C 69/003

(54) **USE OF (Z)-3-HEXENYL ESTERS AND METHOD FOR PROTECTING PLANTS AGAINST PESTS**

(30) Priority: 21.04.2020 ES 202030330
(71) Applicant: Universitat Politècnica de València, 46022 Valencia (ES); Instituto Valenciano De Investigaciones Agrarias (IVIA), 46113 Moncada (Valencia) (ES); Consejo Superior de Investigaciones Científicas (CSIC), 28006 Madrid (ES)
(72) Inventor: PÉREZ HEDO, Meritxel, 46113 Moncada, Valencia (ES); URBANEJA GARCÍA, Alberto, 46113 Moncada, Valencia (ES); ALONSO VALIENTE, Miquel, 46113 Moncada, Valencia (ES); NAVARRO LLOPIS, Vicente, 46022 Valencia (ES); VACAS GONZÁLEZ, Sandra, 46022 Valencia (ES); RAMBLA NEBOT, José Luis, 28006 Madrid (ES); GRANELL RICHART, Antonio, 28006 Madrid (ES)
(74) Representative: Pons
(86) International application number: PCT/ES2021/070259
(87) International publication number: WO 2021/214360

(57) **Abstract**

The present invention relates to the use of esters acetate, propionate and (Z)-3-hexenyl butyrate, or of a composition comprising these compounds, to protect plants, in particular agricultural crops, against pests, by stimulating the defense mechanisms of the plants themselves. The invention also relates to a method for protecting plants against pests by bringing said esters into contact with the plants.

## Description

The present invention relates to the use of (Z)-3-hexenyl esters (acetate, propanoate, or butanoate), or of a composition comprising said compounds to protect plants, particularly agricultural crops, against pests, by stimulating the defense mechanisms that the plants themselves have. The invention also relates to a method for protecting plants against pests by applying said esters to plants.

Therefore, the present invention is comprised in the technical sector of agricultural pest control.

### BACKGROUND OF THE INVENTION

Ensuring food security is one of the most pressing challenges facing the world population today. World agricultural production faces the challenge of meeting the growing demands for food for a population that, according to the FAO, will reach 9,000 million inhabitants in 2050. This need must be addressed despite the adversities that change in consumption patterns, the impacts of climate change, and the growing scarcity of water and arable land may bring. Furthermore, in addition to these adversities are the already significant crop yield losses caused by different abiotic and biotic stresses.

In the particular case of pests, pathogens, and weeds, crop losses vary with each particular crop, but in general, it is accepted that pests and diseases participate in a similar way, with 15% of each group, while weeds do so with another 13%. Adding 9 and 20% of additional post-harvest losses to these amounts is necessary.

Unfortunately, these figures will increase in the coming years due to the new conditions resulting from climate change, such as the introduction of new pests or exotic diseases in our agriculture. By way of example, in the Community of Valencia, the number of exotic agricultural pests introduced over the past 20 years amounts to almost one per year. Sometimes they are introductions that can go unnoticed, but unfortunately, some heavily invade crops in that Community as they are not accompanied by their natural enemies, quickly becoming key pests. A case known due to the media coverage it entailed, and that can serve as a paradigmatic example of an exotic plague in said Community, is the tomato moth, *Tuta absolute* (Meyrick) (Lepidoptera: Gelechiidae), which was detected at the end of 2006 in the province of Castellón and quickly became a key pest of this crop throughout the world.

From the birth of agriculture some 10,000 years ago to just 65 years ago, agriculture was a holistic activity based on a systemic approach. Agricultural societies designed crop protection programs based primarily on pest prevention. This proper form of agroecological management included, among other things, crop rotation, planning different crop combinations, use of resistant or tolerant cultivars, choice of correct sowing and harvesting periods, biological control, mechanical control, physical control, etc.

However, as plant genetics, synthetic fertilizers, and pesticides were developed, agricultural research changed from a holistic approach to a highly reductionist science where pests were combated mainly based on treatment schedules or curative treatments. The increasing use of synthetic pesticides has led to a severe loss of biodiversity, resulting in a reduction in the functioning of the ecological infrastructures that regulate pests, pollination, and water purification. Furthermore, it has also meant an exorbitant contribution of resources and financial consequences amounting to billions of Euros.

At this point, in recent years and from various governments and organizations worldwide, a call has been made to return to a more ecological approach to crop protection. Throughout the European Union, a framework for community action has been established to achieve sustainable use of pesticides (Directive 2009/128/EC), which has led to the obligation that all fruit and vegetable production in the European Union be under the precepts of Integrated Pest Management (IPM) (or, where applicable, organic farming).

Despite all these efforts, currently both in IPM and organic farming, unfortunately, in most cases, pest control still relies on curative treatments with pesticides since there are products authorized for it in both types of agriculture. In the context in which this invention is presented, it is understood that regardless of the kind of pest management used (IPM or ecological), the first line of crop protection should be the implementation of preventive control methods such as cultural methods, use of resistant/tolerant plants, the application of strict quarantine regulations, and the promotion of natural pest control. In this previous context, the present invention's use would fit. Despite these efforts, pests exceed or are expected to exceed acceptable population levels (economic damage threshold), biological pest control should be the first option to use, if necessary, in combination with other IPM tactics (where pesticides would be last).

This concept, which has lately been coined "conscious farming", would respect the environment and the availability of resources for future generations.

In recent years, the use of omnivorous natural enemies in horticultural crops, and in particular zoophytophagous predators that can feed both on the plant and on prey, has given rise to some of the most resounding successes of biological control in Spain. In that sense, for example, in pepper, the release and conservation of the predatory mite *Amblyseius swirskii* (Athias-Henriot) (Acari: Phytoseiidae) together with the anthocorid *Orius laevigatus* (Fieber) (Hemiptera: Anthocoridae) makes it possible to successfully manage the populations of the key pests of this crop: the whitefly *Bemisia tabaci Gennadius* and the thrip *Frankliniella occidentalis* (Pergande) (Thysanoptera: Thripidae). Similarly, in tomato, the cosmopolitan plant bug *Nesidiocoris tenuis* (Reuter) (Hemiptera: Miridae) allows effective control of *B. tabaci* and the tomato moth *T. absolute.*

Recently, it has been shown how some of these zoophytophagous predators, due to their phytophagy, activate the same defense mechanisms that are caused by obligate herbivorous arthropods. It is widely known that plants respond to phytophagous attacks (induced defenses) through various response pathways.

These defenses can, among other phenomena, cause the production of secondary metabolites and proteins that have toxic effects, repellents, and/or antifeedants on herbivores (direct defenses). They can also trigger the production and release of volatiles (herbivore-induced plant volatiles: HIPVs) by the plant, which can modify the behavior of both phytophagous pests and their natural enemies (indirect defenses). Previous works cited below have shown that several species of zoophytophagous predators used in biological pest control strategies can induce defenses in various horticultural crops such as peppers and tomatoes. These inducible defenses are undoubtedly an added value that these biological control agents possess and, if properly managed, could offer an excellent tool to increase the resilience of crops.

In the first stage, it was possible to verify how the phytophagy of the predator *N. tenuis* activated the abscisic acid and jasmonic acid (JA) metabolic pathway in tomato plants, which made them less attractive to the whitefly *B. tabaci,* and more attractive to the whitefly parasitoid *Encarsia formosa* (Gahan) (Hymenoptera: Aphelinidae). Furthermore, it was observed how the volatiles emitted by plants stung by *N. tenuis* could induce defenses in neighboring intact plants by activating the JA pathway, which also resulted in the attraction of parasitoids by these intact plants that had not been exposed to *N. tenuis.* Later it was confirmed that all stages of development of *N. tenuis* (from young nymphs to adults) are capable of triggering these defensive responses. However, not all zoophytophagous predators have the same ability to induce such responses in tomato plants. Tomato plants can have different degrees of attraction for pests and natural enemies depending on whether phytophagy is produced, for example, by *N. tenuis, Macrolophus pygmaeus* (Rambur), or *Dicyphus maroccanus Wagner* (Hemiptera: Miridae). In that sense, while plants damaged by *N. tenuis* reject *B. tabaci* and *T. absolute,* the phytophagy of *M. pygmaeus* and *D. maroccanus* has no effect on repellency in *B. tabaci* and attracts *T. absolute.* In contrast, the activity of the three plant bugs results in the attraction of *E. formosa.*

Volatiles (HIPVs) involved in the defensive responses of tomato plants induced by *M. pygmaeus* and *N. tenuis* have been identified: six green leaf volatiles (GLVs), methyl salicylate and octyl acetate ((Pérez-Hedo, M. et al. Biocontrol, 63: 203-213 (2018)). In general, plants exposed to *N. tenuis* emitted more volatiles than plants exposed to *M. pygmaeus,* and the latter emitted more volatiles than the intact plants. The six GLVs and the methyl salicylate turned out to be repellent to *B. tabaci* and attractive to *E. formos*a*,* while they showed no effect on *T. absolute.* These results clearly show how plant bug herbivory can modulate the preference of a pest or a natural enemy based on exposure to a particular volatile and opens the door to possible practical applications of these compounds.

In pepper, it has been verified how the phytophagy of the anthocorid *O. laevigatus* triggers defensive responses in the plant (Bouagga et al. Journal of Pest Science, 91: 55-64 (2018)). Specifically, pepper plants exposed to *O. laevigatus* induce repellency against the whitefly *B. tabaci* and the thrip *F. occidentalis.* On the contrary, the whitefly parasitoid *E. formosa* is attracted to plants exposed to *O. laevigatus.* The phytophagy of *O. laevigatus* triggers the release of a mixture of volatiles (5 terpenes, 2 GLVs, methyl salicylate, and one to be identified) and the activation of the jasmonic acid and salicylic acid metabolic pathways. Similarly, the phytophagy of plant bugs *N. tenuis* and *M. pygmaeus* in pepper also caused repellency for *B. tabaci* and *F. occidentalis* and attraction to *E. formosa* and triggered volatiles similar in nature to those triggered by *O. laevigatus.*

Perhaps one of the most exciting results to date has been the verification that in greenhouse tests on both tomato and pepper plants that had previously been activated (exposed) for 24 hours by *N. tenuis,* the infestation of the two-spotted spider mite, *Tetranychus urticae Koch* (Acari: Tetranychidae), and of *B. tabaci* was significantly smaller compared to pepper plants not exposed to the predator. Since zoophytophagous predators have been used in horticultural crops, a lower incidence of certain viruses has been observed. It has recently been verified how the defenses induced by predatory plant bugs decrease the multiplication of phytopathogenic viruses, specifically tomato spotted wilt virus on pepper (TSWV) (Bouagga et al. Pest Management Science, 76: 561-567 (2020)). As previously mentioned, volatile compounds have been identified that are responsible for the repellency and attraction of pests and natural enemies.

In recent years, the number of research projects attempting to use these volatiles to induce plant defenses has increased considerably (Turlings, T.C.J., Erb, M. Annu. Rev. Entomol. 63, 433-452 (2018)). For example, in corn, exposure to (Z)-3-hexenal, (Z)-3-hexen-1-ol, and (Z)-3-hexenyl acetate managed to overexpress the jasmonic acid pathway (Engelberth, J., H. T. Alborn, E. A. Proc. Natl. Acad. Sci. 101, 1781-1785 (2004). However, none of them has been able to obtain results in which the defensive response of plants is seen in real field conditions. In contrast, the application of (Z)-3-hexenyl propionate using dispensers has caused a defensive response in commercial crops, as presented in this invention.

This invention shows that (Z)-3-hexenyl esters are elicitors of the induction of direct and indirect defenses in crops, such as the tomato crop. Specifically, it has been possible to activate the plants defensively simply by exposing them to these volatiles of synthetic origin. The exposure of (Z)-3-hexenyl esters has been described as being repellent to the phytophagous pest, the whitefly, *Bemisia tabaci,* and attractant to natural enemies such as the whitefly parasitoid, *Encarsia formosa* (Pérez-Hedo, M. et al. Biocontrol, 63: 203-213 (2018)). The attraction to parasitoids is an indirect effect that can benefit the biological control of a pest.

The present invention is a sustainable and biorational pest control tool based on communication between plants for crop protection. The plants use the emission of these volatiles to communicate certain aggressions to their "neighbors". The plants receiving these signals enter a state of alert to prepare for a future attack. This is known as priming.

### DESCRIPTION OF THE INVENTION

The inventors of the present invention have found that the compounds according to Formula I, wherein R=CH₃, CH₂-CH₃ or CH₂-CH₂-CH₃, are capable of alerting plants exposed to the same and inducing defense mechanisms in such plants that reduce the impact of pests in such a way that it induces or elicits the natural defenses of plants.

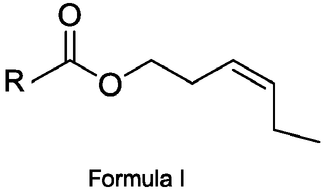

Using these volatile compounds, the plant is activated defensively through the hormonal pathways of jasmonic acid and salicylic acid, both phytohormones involved in plant defense mechanisms.

The defensive activation achieved with these compounds makes the activated plants more resistant to pest attack.

Then, in a first aspect, the present invention relates to the use of at least one compound of formula I defined above, or of a composition comprising the same, for the protection of plants against pests by stimulating the natural defense mechanisms of said plants.

These mechanisms make plants repel pests and/or attract parasitoids of said pests. These parasitoids are natural enemies of pests, so, accordingly, their attraction means that the plants are protected against the pests mentioned above.

The compounds used in the present invention are (Z)-3-hexenyl acetate, propanoate, or butanoate. In a preferred embodiment, the invention relates to the use of (Z)-3-hexenyl propanoate, or of a composition comprising same, for the protection of plants against pests by stimulating the natural defense mechanisms of said plants.

In a preferred embodiment, the defense mechanisms of the plants cause the repellency of at least one pest selected from the list comprising: *Bemisia tabaci* (Gennadius), *Trialeurodes vaporariorum* (Westwood), *Aleurothrixus floccosus* (Maskell), *Dialeurodes citri* (Ashmead) and *Paraleyrodes minei* laccarino (Hemiptera: Aleyrodidae), the thrips *Frankliniella occidentalis* Pergande, *Pezothrips kellyanus* Bagnall, *Chaetanaphothrips orchidii* (Moulton) and *Thrips tabaci* Lindeman (Thysanoptera: Thripidae), the spider mites *Tetranychus urticae, Eutetranychus orientalis* (Klein), *Eutetranychus banksi* (McGregor), *T. urticae, Panonychus citri* (McGregor) Koch and *Tetranychus evansi* Baker & Pritchard (Acari: Tetranychidae), the lepidopterans *Tuta absolute* (Meyrick), *Phyllocnistis citrella* Stainton (Lepidoptera: Gracillariidae), *Spodoptera exigua* Hübner and *Helicoverpa armigera* (Hübner) (Lepidoptera: Noctuidae) in horticultural crops; and the psyllids *Diaphorina citri* Kuwayama (Hemiptera: Liviidae) and *Trioza erytreae* (Del Guercio) (Hemiptera: Psyllidae), in citrus crops.

In an even more preferred embodiment, the pest is selected from the list comprising: the whitefly *Bemisia tabaci* (Gennadius) (Hemiptera: Aleyrodidae), the thrips *Frankliniella occidentalis* Pergande (Thysanoptera: Thripidae), the spider mite *Tetranychus urticae* Koch (Acari: Tetranychidae), the lepidopteran *Tuta absolute* (Meyrick) (Lepidoptera: Gelechiidae) and the psyllid *Diaphorina citri.*

In a preferred embodiment, the defense mechanisms of the plants cause the attraction of at least one parasitoid selected from the list comprising: species of the genera *Encarsia* spp., *Aphytis* spp. *Cales* spp., *Eretmocerus* spp., *Aphelinus* spp., (Hymenoptera: Aphelinidae), *Aphidius* spp. *Lysiphlebus* spp. (Hymenoptera: Braconidae) *Metaphycus* spp., *Anagyrus* spp. (Hymenoptera: Encyrtidae), *Tamarixia* spp. *Citrostichus* spp., *Cirrospilus* spp., *Diglyphus* spp. (Hymenoptera: Eulophidae), *Trissolcus* spp., *Telenomus* spp. (Hymenoptera: Scelionidae) and *Trichogramma* spp. (Hymenoptera. Trichogrammatidae).

The aforementioned compound has been shown to repel pests of horticultural crops from different orders of insects (Hemiptera, Thysanoptera and Lepidoptera), which was not to be expected precisely because they are very phylogenetically separated species. In the case of citrus fruits, it has been observed how activation with this same volatile decreases the laying of the psyllid *Diaphorina citri.*

In a preferred embodiment, the protected plants are horticultural plants or citrus plants. Horticultural plants are preferably selected from the list comprising: tomatoes, peppers, eggplants, onions, leeks, pumpkins, zucchinis, cucumbers, chard, lettuce and legumes. More preferably, horticultural plants are tomato plants (*Solanum lycopersicum*) or pepper plants (*Capsicum annuum*)*.* The citrus fruits are preferably selected from the list comprising lemons, limes, oranges, grapefruits, and tangerines.

In a more particular embodiment, the plant is a tomato plant, and the pest is the whitefly *B. tabaci,* the thrips *Frankliniella occidentalis* or the lepidopteran *Tuta absoluta.*

In another particular embodiment, the plant is a citrus plant and the pest is the psyllid *Diaphorina citri.*

In a preferred embodiment, the use comprises the application of the compounds according to Formula I using dispensers, which allows the effective use of said compounds via exposure under field conditions as an inducer/elicitor of plant defenses. This emission system, using dispensers, manages to obtain, in the atmosphere surrounding the plants to be protected, a sustained and sufficient concentration of the inducer over time that would not be obtained with an isolated application.

According to the present invention, a "dispenser" (also called "emitter") is understood as a device or carrier (formulation) that distributes at a controlled rate at least one of the compounds of the invention (Formula I) via exposure thereof to plants under field conditions. The devices are containers for the compounds according to Formula I that allows said substances to be released into the atmosphere in a controlled manner through a membrane, a valve or even through the container wall itself. Carriers are formulations of liquid, gelled or pasty substances that contain the compounds according to Formula I and are applied in the form of drops or granules so that the volatiles are emitted as the carrier degrades or dehydrates. These formulations or carriers can be applied to any part of the plant, substrate, crop structures, or place from which it can be emitted close to the plants. Among these carriers are substances such as paraffins, silicones, pectins, heavy oils, polysaccharides, proteins, etc. Among them, the following can be mentioned by example: agar-agar, alginine, carrageenan, collagen, corn starch, gelatin, guar gum, locust bean gum, pectin, xanthan gum, mineral oil, silicone oil, paraffins, olefins or mineral or vegetable oils.

Dispensers are preferably devices of two types:
(1) passive emitters consisting of a container for the compounds according to Formula I configured to allow the diffusion of said substances, so the container must be permeable to the compounds of formula I, either through its enclosure of a polymeric material or through a membrane placed in a part of its enclosure to regulate the emission;
(2) active emitters in which the compounds according to Formula I are packaged in a pressure vessel containing a valve configured for being actuated in a programmed manner, as is the case with nebulizers that produce an aerosol.

In a more preferred embodiment, the compounds according to Formula I are applied using dispensers consisting of vials made of low-density polyethylene (LDPE).

In a second aspect, the present invention relates to a method for the protection of plants against pests by stimulating the natural defense mechanisms of said plants, said method is characterized in that it comprises contacting at least one compound of Formula I or a composition comprising same with the plants. Contact can be direct using direct application of the compound to the plant or indirect by means of exposing the plant to an environment containing said compound.

These natural defense mechanisms of plants allow them to repel pests or attract their natural parasitoids.

In a preferred embodiment of the method, it is characterized in that it comprises the application of (Z)-3-hexenyl propanoate or a composition comprising the same.

In a preferred embodiment of the method, the pest is selected from the list comprising: *Bemisia tabaci* (Gennadius) and *Trialeurodes vaporariorum* (Westwood), *Aleurothrixus floccosus* (Maskell), *Dialeurodes citri* (Ashmead) and *Paraleyrodes minei* laccarino (Hemiptera: Aleyrodidae), the thrips *Frankliniella occidentalis* Pergande, *Pezothrips kellyanus* Bagnall, *Chaetanaphothrips orchidii* (Moulton) and *Thrips tabaci* Lindeman (Thysanoptera: Thripidae), red spiders *Tetranychus urticae, Eutetranychus orientalis* (Klein), *Eutetranychus banksi* (McGregor), *T. urticae, Panonychus citri* (McGregor) Koch and *Tetranychus evansi* Baker & Pritchard (Acari: Tetranychidae), lepidoptera *Tuta absolute* (Meyrick), *Phyllocnistis citrella* Stainton (Lepidoptera: Gracillariidae), *Spodoptera exigua* Hübner and *Helicoverpa armigera* (Hübner) (Lepidoptera: Noctuidae) in horticultural crops; and psyllids *Diaphorina citri* Kuwayama (Hemiptera: Liviidae) and *Trioza erytreae* (Del Guercio) (Hemiptera: Psyllidae), in citrus fruits.

In an even more preferred embodiment, la the pest is selected from the list comprising: the white fly *Bemisia tobaci* (Gennadius) (Hemiptera: Aleyrodidae), the thrip *Frankliniella occidentalis* Pergande (Thysanoptera: Thripidae), the red spider *Tetranychus urticae* Koch (Acari: Tetranychidae), the lepidoptera *Tuta absoluta* (Meyrick) (Lepidoptera: Gelechiidae) and the psyllid *Diaphorina citri.*

In a preferred embodiment of the method, plant protection is produced by the attraction of parasitoids selected from the list comprising: species of the genera *Encarsia* spp., *Aphytis* spp. *Cales* spp., *Eretmocerus* spp., *Aphelinus* spp., (Hymenoptera: Aphelinidae), *Aphidius* spp. *Lysiphlebus* spp. (Hymenoptera: Braconidae) *Metaphycus* spp., *Anagyrus* spp. (Hymenoptera: Encyrtidae), *Tamarixia* spp. *Citrostichus* spp., *Cirrospilus* spp., *Diglyphus* spp. (Hymenoptera: Eulophidae), *Trissolcus* spp., *Telenomus* spp. (Hymenoptera: Scelionidae) and *Trichogramma* spp. (Hymenoptera. Trichogrammatidae).

In a preferred embodiment of the method, the protected plants are horticultural plants or citrus plants. Horticultural plants are preferably selected from the list comprising: tomatoes, peppers, eggplants, onions, leeks, pumpkins, zucchinis, cucumbers, chard, lettuce and legumes. More preferably, horticultural plants are tomato plants (*Solanum lycopersicum)* or pepper plants (*Capsicum annuum*)*.* The citrus fruits are preferably selected from the list comprising lemons, limes, oranges, grapefruits and tangerines.

In a more particular embodiment, the plant is a tomato plant and the pest is the whitefly *B. tabaci,* the thrip *Frankliniella occidentalis* or the lepidoptera *Tuta absoluta.*

In another particular embodiment, the plant is a citrus plant and the pest is the psyllid *Diaphorina citri.*

In a preferred embodiment of the method, the compounds according to Formula I, or of a composition comprising the same, are applied by means of the use of dispensers. Dispensers and preferred embodiments according to the present invention have been defined in the first aspect of the invention. In a more preferred embodiment, the compounds according to Formula I are applied using dispensers consisting of low-density polyethylene vials.

In a preferred embodiment, the application is carried out at the rate of one dispenser for every 20 m² of the planted surface. In this way, the dispenser distributes the compound into the environment surrounding the plants, and such plants detect it.

In a preferred embodiment of the method, the compounds according to Formula I are applied to the crop environment at a dose between 25 mg/ha/day and 25 g/ha/day, and more preferably between 200 mg/ha/day and 10 g/ha/day.

During the development of this invention, it has been observed, by means of the use of different dispensers, how the plant is defensively activated when the volatile emitting sources do so from 25 mg/ha/day of the compounds according to Formula I. It has been verified how a passive polymeric dispenser can release, depending on the environmental conditions, a dose of the volatile in the range sufficient to activate tomato, pepper, or citrus plants under field conditions.

The development of this invention allows for the first time to defensively activate plants such as tomato, pepper, and citrus plants and make them more resilient against pests by exposing plants to volatiles, according to Formula I.

The methodology described in this application by exposure to the compounds according to Formula I activates a previously undescribed response in the plant related to the overexpression of the salicylic acid and jasmonic acid pathways. This activation causes the plants to emit volatiles that attract and repel natural enemies and pests. In the case of tomato, it has been observed that plants exposed to (Z)-3-hexenyl propanoate repel key pests of this crop, such as the whitefly, *B. tabaci,* the thrip *F. occidentalis* and the lepidopteran *T. absoluta.* This activation, therefore, allows repelling of three key pests, which was not expected since these three species belong to three different orders of insects (Hemiptera, Thysanoptera and Lepidoptera). In the case of citrus fruits, it has been observed how activation with this same volatile repels the psyllid *D. citri.* It has been shown in the case of citrus fruits that the laying of *D. citri* is also reduced by more than half when the citrus plant has been exposed to this volatile. Plants activated by exposure to (Z)-3-hexenyl propanoate are also more attractive to natural enemies of pests such as parasitoids. *Encarsia formosa* in tomato and *Tamarixia radiata* in citrus fruits.

In the development of the present invention, it has also been observed how the defensive activation of the plant does not affect the establishment or development of zoophytophagous predators that may colonize said crop.

Contrary to what might be expected, the overexpression of defensive pathways in plants caused by exposure to the volatile (Z)-3-hexenyl propanoate does not reduce the growth or production of the plant, obtaining, as a final result, plants that are just as productive but more resistant to pest attack.

The present invention is a new pest management method not used to date in any crop.

Throughout the description and the claims, the word "comprises" and its variants do not intend to exclude other technical features, additives, components, or steps. For those skilled in the art, other objects, advantages, and features of the invention may be partially deduced from both the description and the embodiment of the invention. The following examples and figures are provided by way of illustration and are not intended to limit the present invention.

### BRIEF DESCRIPTION OF THE FIGURES

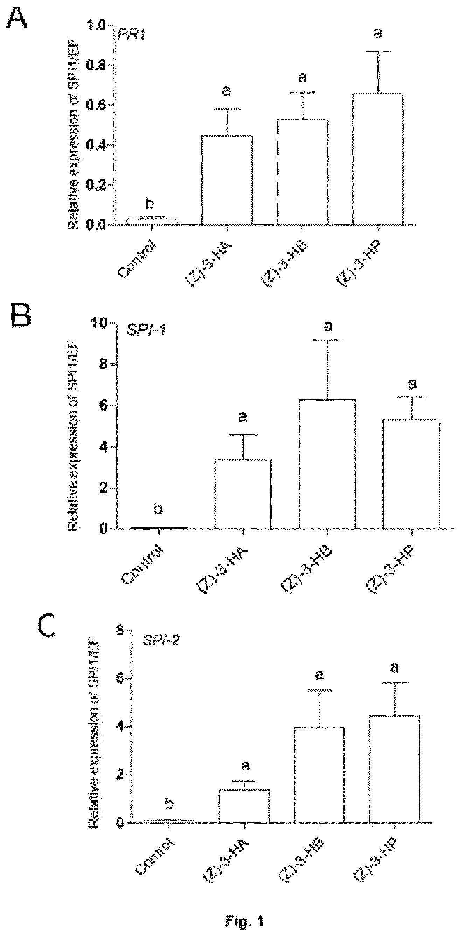

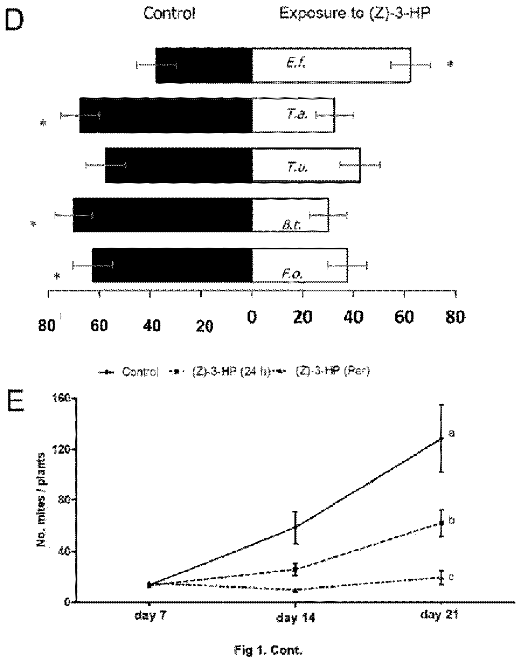

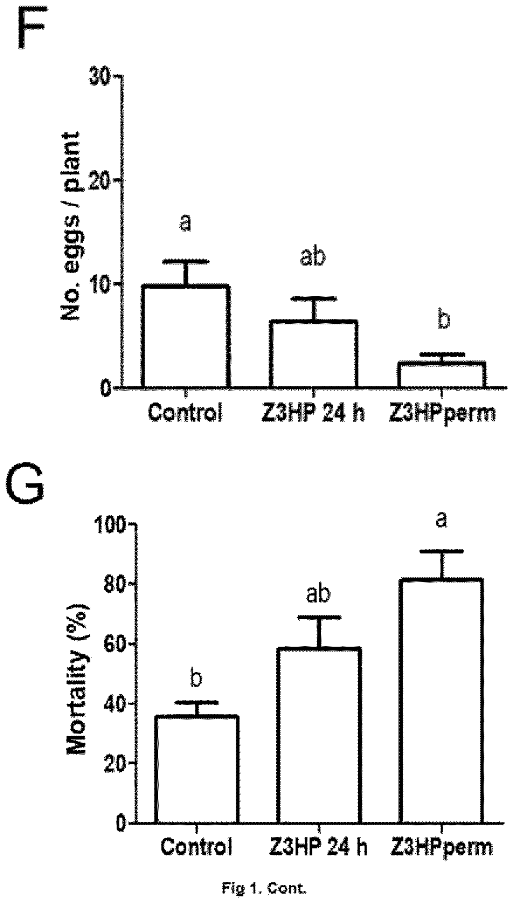

**Figure 1****.:** Transcriptional response of defense genes PR1 (a marker gene for the salicylic acid signaling pathway, SA) **(****Figure 1A****)** and SI-PI-I and SI-PI-II (two markers for plant proteinase inhibitors) (Figure **1B and 1C****,** respectively) in tomato plants exposed to (Z)-3-hexenyl acetate (HA), (Z)-3-hexenyl butanoate (HB) and (Z)-3-hexenyl propanoate (HP) and control. Data are presented as the mean of eight independent analyses of transcript expressions relative to a housekeeping gene ± standard error (n = 8). Significant differences based on the ANOVA test are marked with different letters (P < 0.05). Response (% ± SE (Standard Error)) of female *Encarsia formosa* (*Ef.*)*, Tuta absoluta* (*T.a.*)*, Tetranychus urticae* (*T.u.*)*, Bemisia tabaci* (*B.t.*) and *Frankliniella occidentalis (F.o.)* in a Y-tube olfactometer when exposed to the control (1: 10,000 methanol: water) and to (Z)-3-hexenyl propanoate ((Z)-3-HP) (1: 10,000 with the volatile to test). Asterisks indicate significant differences in the distribution of side arm choices (X² tests; P < 0.05) **(****Figure 1D****).** Number (mean ± standard error) of *Tetranychus urticae* females per tomato plant when comparing mite development in tomato plants exposed to (Z)-3-hexenyl propanoate [(Z)-3-HP)] compared to unexposed tomato plants (Control). Exposure to (Z)-3-HP was tested in two types of bioassays, (24 h) and (Per): in the first type, the plants were only exposed for 24 hours to the volatile before the release of *T*. *urticae* (24 h); In the other type of test, the plants were permanently exposed to the volatile throughout the experiment (Per). Bars with different letters are significantly different (GLMM, repeated measures α < 0.05) **(****Figure 1E****).** Number of eggs (mean ± standard error) laid by 2 *Tuta absolute* females for 72 hours in plants exposed to (Z)-3-hexenyl propanoate [(Z)-3-HP)] compared to unexposed tomato plants (Control). Bars with different letters are significantly different (ANOVA, Tukey α < 0.05) **(****Figure 1F****).** Mortality percentage (mean ± standard error) of *T. absolute* from egg to adult when developed in tomato plants exposed to (Z)-3-hexenyl propanoate [(Z)-3-HP)] compared to untreated tomato plants (Control). Bars with different letters are significantly different (ANOVA, Tukey α < 0.05) **(****Figure 1G****).**

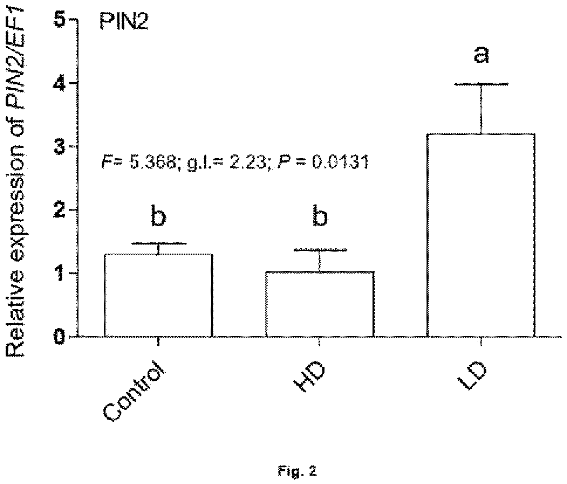

**Figure 2****.** Transcriptional response of the PIN2 gene (a marker gene for the jasmonic acid (JA) signaling pathway in tomato plants exposed under semi-field conditions to (Z)-3-hexenyl propanoate [(Z)-3-HP] with different volatile emission levels: 5 mg/day/ dispenser in the low-density (LD) polyethylene emitter and 0.01 mg/day/ dispenser in the high-density (HD) polyethylene emitter. The control treatment consists of plants not exposed to the volatile. Significant differences based on the ANOVA test are marked with different letters (P < 0.05).

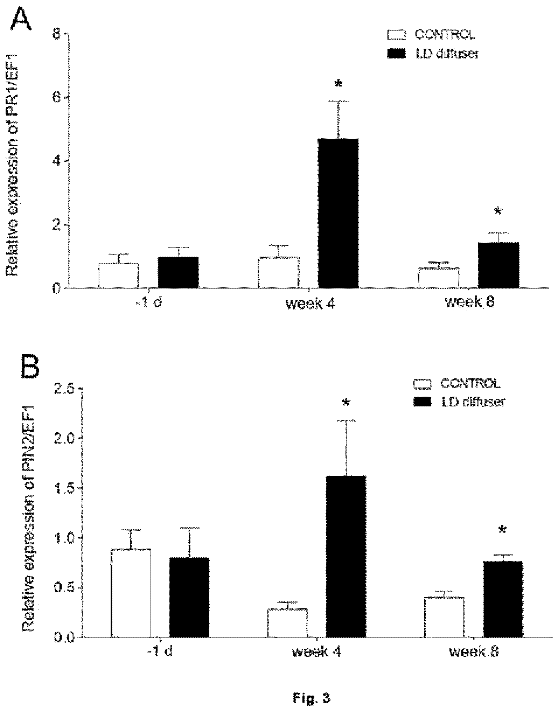

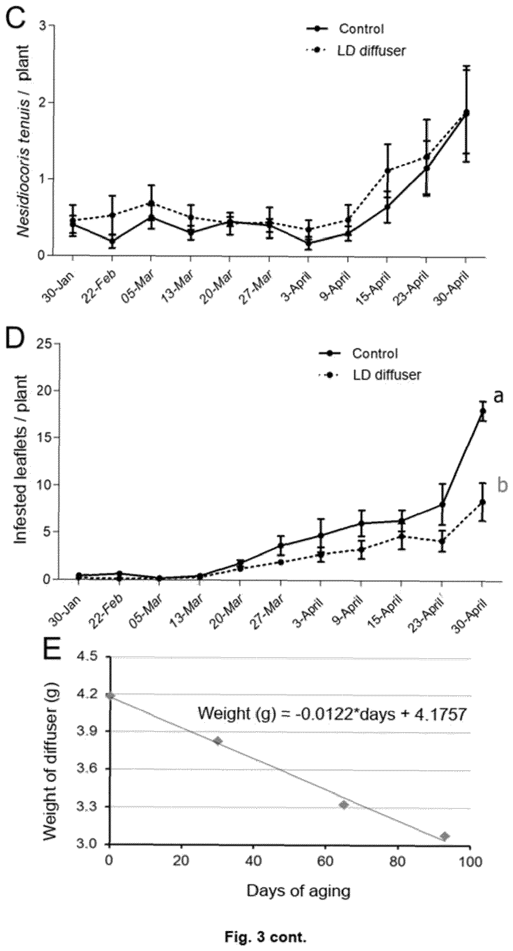

**Figure 3****:** Transcriptional response of defense genes PR1 (a marker gene for the SA signaling pathway) **(****Figure 3A****)** and PIN2 (a marker gene for the jasmonic acid signaling pathway) **(****Figure 3B****)** in tomato plants exposed under field conditions to (Z)-3-hexenyl propanoate [(Z)-3-HP] released in a 4-mL low-density (LD) polyethylene polymeric dispenser vial (Kartell) a day before, 4 and 8 weeks after the placement of the dispenser. Significant differences between treatments for each date are marked with an asterisk (t-test; P < 0.05). Number of *Nesidiocoris tenuis* per plant (mean ± standard error) **(****Figure 3C****)** and number of infested leaflets per plant (mean ± standard error) **(****Figure 3D****)** in the commercial greenhouse (GLMM, P < 0.05). Release profile of the dispenser used fitted from the linear regression model **(****Figure 3E****).**

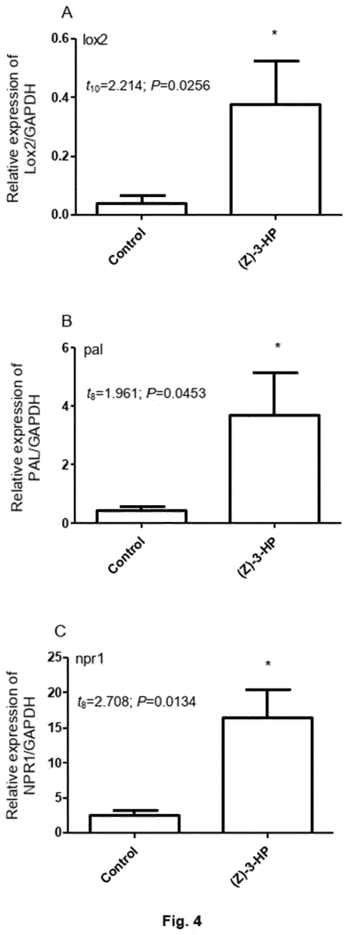

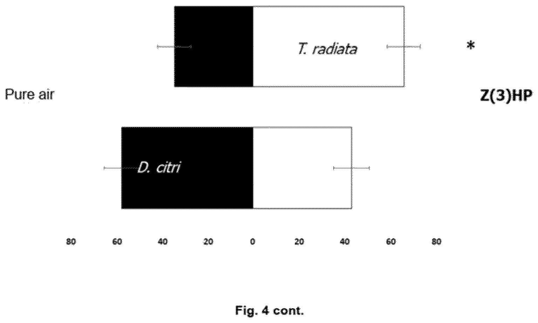

**Figure 4****:** Transcriptional response of defense genes LOX2 (precursor of JA jasmonic acid) **(****Figure 4A****)** and PAL and NPR1, two genes related to the salicylic acid pathway **(****Figure 4B** **and C,** respectively) in Carrizo citrange plants exposed to (Z)-3-hexenyl propanoate and control. Data are presented as the mean of six independent analyses of transcript expressions relative to a housekeeping gene ± standard error (n = 6). Significant differences based on the *t*-test are marked with (*) (P < 0.05). Response (% ± SE) of *Diaphorina citri* y *Tamarixia radiata* females in a Y-tube olfactometer when exposed to control (1: 10,000 methanol: water) and to (Z)-3-hexenyl propanoate ((Z)-3-HP) (1: 10,000 with the volatile to test). Asterisks indicate significant differences in the distribution of side arm choices (*X*² tests; P < 0.05) **(Figure 4D).**

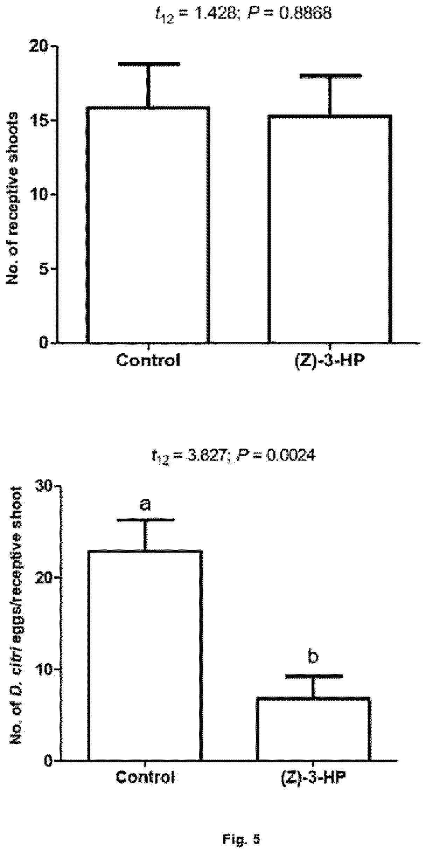

**Figure 5****:** Number of receptive shoots (mean ± SE) and laying (mean ± SE) of *Diaphorina citri* in the seedlings of *Murraya paniculata* used as sentinel plants with and without a dispenser loaded with HP. Different letters on the bars show significant differences (*t-*test P < 0.05).

### EXAMPLES

Next, the invention will be illustrated using assays carried out by the inventors.

### Example 1: Defensive activation assays in tomato via exposure under laboratory conditions

### Methodology

To demonstrate that the exposure of volatiles (Z)-3-hexenyl propanoate, (Z)-3-hexenyl acetate, and (Z)-3-hexenyl butanoate activates plant defenses, *Solanum lycopersicum* cv. Moneymaker tomato plants were used. Two weeks after seed germination, the seedlings were individually transplanted into pots (8 × 8 × 8 cm). The plants were kept at 25 ± 2 °C, with a constant relative humidity of 65% ± 5% and a 14:10 h (light: dark) photoperiod. Tomato plants that had not received any pesticide treatment at four weeks of age (about 20 cm tall) were used. All volatiles used were obtained from Sigma-Aldrich (St. Louis, MO, USA). Each volatile was exposed by using a 2 x 2 cm piece of filter paper on which 10 µl of a solution containing the corresponding volatile at a concentration of 1:10,000 [(Z)-3- hexenyl ester: methanol] was impregnated as an emitting source of the corresponding volatile (Pérez-Hedo, M. et al. Biocontrol, 63: 203-213 (2018)).

To activate a plant, two pieces of filter paper impregnated with the volatile were placed at the bottom of a 30 × 30 × 30 cm plastic cage (BugDorm-1 Insect Tents; MegaView Science Co., Ltd, Taichung, Taiwan) into which the plant was introduced. A control treatment was carried out in which the two pieces of filter paper were impregnated with 10 µl of methanol. Eight repetitions per treatment were performed. The plants of the treatments were kept exposed to the volatile without disturbing for 24 hours in separate and isolated climatic chambers to avoid any interference from the volatiles and were kept at 25 ± 2 °C, 65 ± 10% RH, and a 14:10 h (L: D) photoperiod. After 24 hours, the expression of 3 genes related to plant defensive activation was studied in both treatments: the precursor of the protein related to basic pathogenesis (PR-1), a marker gene for the SA signaling pathway, and two markers for plant proteinase inhibitors (SI-PI-I and SI-PI-II) were studied.

The methodology followed for extracting and quantifying gene expression was described by Pérez-Hedo et al. Journal of Pest Science 3: 543-554 (2015). The primers used were forward primer: 5'- CTCATATGAGACGTCGAGAAG-3' (SEQ ID NO: 1) and reverse primer: 5'-GGAAACAAGAAGATGCAGTACTTAA -3' (SEQ ID NO: 2) for the quantification of PR1, forward primer: 5'-TGAAACTCTCATGGCACGAA-3' (SEQ ID NO: 3) and reverse primer: 5'- TTTTGACATATTGTGGCTGCTT-3' (SEQ ID NO: 4) for SI-PI-I, and forward primer: 5'-GGCCAAATGCTTGCACCTTT-3' (SEQ ID NO: 5) and reverse primer: 5'-CAACACGTGGTACATCCGGT-3' (SEQ ID NO: 6) for the SI-PI-II gene. The nucleotides of the constitutive gene EF1 were forward primer: 5'-GATTGGTGGTATTGGAACTGTC-3' (SEQ ID NO: 7) and reverse primer: 50-AGCTTCGTGGTGCATCTC-30 (SEQ ID NO: 8).

After verifying that exposure to the three volatiles activated genes related to plant defenses, (Z)-3-hexenyl propanoate was selected from among the three volatiles as a model for the (Z)-3-hexenyl esters.

It was studied whether the defensive activation achieved by exposure to (Z)-3-hexenyl propanoate could affect repellency and/or attraction in herbivores that attack tomatoes, the whitefly *Bemisia tobaci* (Gennadius) (Hemiptera: Aleyrodidae), the thrip *Frankliniella occidentalis* Pergande (Thysanoptera: Thripidae), the red spider *Tetranychus urticae* Koch (Acari: Tetranychidae) and the tomato borer, *Tuta absoluta* (Meyrick) (Lepidoptera: Gelechiidae), and in a natural enemy that has been used as a model organism in this crop, the parasitoid *Encarsia formosa* Gahan (Hymenoptera: Aphelinidae).

The last instar nymphs of *B. tabaci* and the pupae of *E. formosa* were provided by Koppert Biological Systems, S.L. (Aguilas, Murcia, Spain). The recently emerged B. *tabaci* adults (less than 2 days old) were released into tomato plants inside 60 × 60 × 60 cm plastic cages (BugDorm-2) and placed in a climatic chamber at 25 ± 2 °C, 65 ± 10% RH and a 14:10 h (L: D) photoperiod in IVIA. Five-day-old *B. tabaci* adults were used in all the experiments. In the case of *E. formosa,* the pupae were placed in a Petri dish 9 cm in diameter and allowed to emerge under ambient laboratory conditions (25 ± 2 °C), with a small drop of honey as food. *E. formosa* females less than two days old were used in all the experiments. *F. occidentalis* adults came from a brood kept in captivity established in the Valencian Institute of Agrarian Research (*Instituto Valenciano de Investigaciones Agrarias,* IVIA) in 2010, originally from Campo de Cartagena (Murcia, Spain). The thrips brood was kept in bean pods (*Phaseolus vulgaris* L.; Fabales: Fabaceae) under the same conditions described above. All the *F. occidentalis* females used for experimentation were less than five days old. *T. urticae* adults were obtained from a colony established in IVIA in 2011, which initially came from clementines located in La Plana (Castelló, Spain). The mites were kept on tomato plants in a climatic chamber under the same conditions described above. *T. absoluta* females were obtained from colonies on tomatoes maintained in a greenhouse at IVIA at 25 ± 4 °C, 60 ± 15% RH, and under a natural photoperiod. Newly emerged adult females less than 5 days old were used in all the assays.

To assess the preference of *F. occidentalis, B. tabaci, T. urticae, T. absoluta* and *E. formosa* for tomato plants that were previously exposed for 24 h to (Z)-3-HP relative to intact plants, a Y-shaped olfactometer was used. The plants were exposed to both volatiles as described above using the pieces of filter paper. The olfactometer (Analytical Research Systems, Gainesville, FL) consisted of a 2.4 cm in diameter Y-shaped glass tube with a 13.5 cm long base and two arms, each 5.75 cm long. Both side arms were connected via high-density polyethylene (HDPE) tubing to two identical glass jars (5-liter volume), each of which was connected to an air pump that produced a unidirectional humidified airflow at 150 ml/min (Pérez-Hedo et al. Journal of Pest Science 3: 543-554 (2015)).

For each species, a female was individually introduced into the inlet tube of the olfactometer and observed until she had walked at least 3 cm down one of the arms of choice or until 15 minutes had elapsed. A total of 40 valid replicates were recorded for each species for each pair of odor sources. Each individual was tested only once. Females that did not choose a side arm within 15 minutes were recorded as "no response" and excluded from data analysis. After recording five responses, the Y-tube was rinsed with soapy water, cleaned with acetone, and allowed to dry for 5 minutes. The odor sources were later switched between the left and right side arms to minimize any spatial effect on choice. The two types of plants (intact and induced) were used only once to assess the response of 10 females and were then replaced with new plants. The Y-tube experiment was performed under the following environmental conditions: 23 ± 2°C and 60 ± 10% RH.

It was also studied whether the defensive activation induced by exposure to (Z)-3-hexenyl propanoate could influence the development of two highly important phytophages in tomato cultivation, the red spider, *T. urticae,* and the lepidoptera *T. absolute.* The individuals used in these experiments came from the captive breeding of these species that are kept in the IVIA described above. The exposure time of the plant to the volatile was tested on both phytophages in two different ways, one where the plant was only exposed to the volatile for 24 hours and then the herbivore to be assayed was immediately released, and the other where the volatile was permanently exposed throughout the experiment.

For this purpose, two climatic chambers were used under the same environmental conditions [25 ± 2 °C, 65 ± 5 % HR and 14:10 h (L: D) photoperiod], wherein, to avoid interference between the volatiles, one was assigned to the (Z)-3-hexenyl propanoate treatments and one to the control treatment. In the climatic chamber in which the exposure to (Z)-3-hexenyl propanoate was assayed, there were placed 12 plastic cages (60 cm × 60 cm × 60 cm) (BugDorm-2. MegaView Science Co., Ltd., Taichung, Taiwan), six per treatment tested (24 h of exposure and permanent exposure), while six cages were placed in the climatic chamber where the control treatment was tested. The cages were equally distributed at a distance of one and a half meters between one another. Each cage represented a replica. In each cage, two pieces of impregnated filter paper were placed at the bottom as explained above, both for the treatment with (Z)-3-hexenyl propanoate and for the control. In the treatment where the volatiles were exposed throughout the experiment, the pieces of impregnated filter paper were replaced every two days.

Eight tomato plants (*Lycopersicon esculentum cv. Moneymaker)* were introduced in each cage. In the experiment with *T. urticae,* to prevent the movement of mites from one plant to another, the plants were isolated individually without touching one another or the walls of the cage. Furthermore, the plants were placed on a small brick inside a plastic tray filled with water, and all the pots were painted with a glue band. The plants were artificially infested with *T. urticae* from the laboratory population mentioned above. Twenty *T. urticae* females were released per plant, evenly distributed on all the leaves with the help of a fine brush. Naked eye sampling was carried out seven, 14, and 21 days after the release of *T. urticae,* where the total number of *T. urticae* females in each plant was counted.

Two consecutive experiments were performed to assess the effect of exposure to both volatiles on *T. absolute.* The first experiment studied the effect on the laying of *T. absolute.* The selected eggs laid in the first experiment were later used in the second experiment to study the mortality of immature *T. absolute* specimens developed in plants exposed to (Z)-3-hexenyl propanoate. The same three treatments described above for *T. urticae* were also assayed for *T. absoluta.* The laying of *T. absolute* was assessed in 8 tomato plants (cv. Moneymaker) per treatment. Each of the plants was isolated inside a plastic cage (60 × 60 × 60 cm) (BugDorm-2) and kept in a climatic chamber at 25 ± 2 °C, 65 ± 5 % HR, 14:10 (L: D) h photoperiod following the same assay distribution described for *T. urticae.* Inside each cage (replica), 2 adult pairs were released from *T. absolute* (males and females) and left undisturbed for 72 hours. After this time, *T. absolute* adults were removed, and the number of eggs was counted.

To study the mortality of *T. absolute* in the plants exposed to the 3 treatments described above, 6 *T. absolute* eggs per plant were equally distributed on all the leaves with the help of a fine brush. The eggs used in each treatment came from the corresponding treatment of the first experiment. Mortality of *T. absolute* was assessed in 8 tomato plants (cv. Moneymaker) per treatment. Each of the plants was isolated inside a plastic cage (60 × 60 × 60 cm) (BugDorm-2 insect tents) and kept in a climatic chamber at 25 ± 2 °C, 65 ± 5 % HR, 14:10 (L: D) h photoperiod following the same treatment distribution described above. The plants were left intact until *T. absolute* adults emerged. As soon as the adults began to emerge, they were counted and removed daily from the cages.

### Result

Exposure to the three volatiles (Z)-3-hexenyl propanoate, (Z)-3-hexenyl acetate, and (Z)-3-hexenyl butanoate of intact tomato plants significantly overexpressed the three markers studied compared to the control, finding no differences between them (Figure 1 A,B,C). PR-1: F₁₋₁₉ = 14.09; *P* < 0.0001 SPI-1: *F*₁₋₁₉ = 21.91; *P* < 0.0001 and SPI-2: *F*₁₋₁₉ = 16.28; *P* < *0.0001.*

The activated plants exposed 24 hours to (Z)-3-hexenyl propanoate turned out to be repellent against highly important pests in tomato, such as the lepidoptera *T. absolute* (² = 9.80; *P* = 0.0017), the white fly *B. tabaci* (² = 12.80; *P* = 0.0003) and the thrips *F. occidentalis* (² =5.00; *P* = 0.0253) (Figure 1D). Furthermore, plants exposed to this volatile were more attractive to the parasitoid *E. formosa* (² = 5.00; *P* = 0.0253). The red spider *T. urticae* was the only species that showed no preference (² = 1.80; *P* = 0.1797) (Figure 1D).

In the case of the red spider, 21 days after release, a reduction in the number of mites per plant of 50.3 ± 6.3% and 83.9 ± 5.0% with respect to the control was obtained in the treatments where the plant was kept exposed for 24 h and throughout the entire experiment to (Z)-3-hexenyl propanoate, respectively (*F*_{4.85} = 4.437; *P* = 0.003) (Figure 1E). In the case of *T. absolute,* in the treatment of permanent exposure to (Z)-3-HP, a reduction in laying of 67.2 ± 15.0% was obtained when compared to the control (*F*_{2.23} = 3.746; *P* = 0.0406) (Figure 1F). Furthermore, mortality from *T. absolute* egg to adult in plants permanently exposed to (Z)-3-HP was around 80%, which was significantly higher than the control treatment (*F*_{2.23} = 6,944; *P* = 0.0048) (Figure 1G), the laying (*F*_{2.23} = 1.147; *P* = 0.3367) or in mortality (*F*_{2.23} = 0.5881; *P* = 0.5643) of *T. absolute.*

### Example 2: Semi-field release assays with different application doses of (Z)-3-hexenyl propionate from polymer-type passive dispensers.

### Methodology

Two different types of polymeric emitters were used to test the activation level that different emission levels of (Z)-3-hexenyl propionate can provide: 4-mL low-density (LD) polyethylene vial and 4-mL high-density (HD) polyethylene vial. The highest emission level (5 mg/day) was achieved through previous laboratory studies with LD dispensers loaded with 20 mg of the pure substance. The lowest emission level (0.01 mg/day) was provided by HD vials loaded with a 1:100 mixture of (Z)-3-hexenyl propionate and paraffinic oil (0.02:2; g:g). The emission kinetics of the emitters used during the experiments was controlled by studying weight loss (gravimetric method). Additional dispensers were placed in the same environmental conditions, measuring their weight weekly with a precision scale (0.0001 g). The difference in weight recorded in each period will be the emitted quantity of the substance for each type of dispenser. To obtain the mean emission level of each dispenser, the recorded weights (y) are related to the aging time (x) using a multiple regression analysis.

Once the mentioned emission levels have been established, 5 and 0.01 mg/day/ dispenser, tomato plants were exposed to the different emission levels through an experiment under semi-field conditions in a glass greenhouse. For that purpose, there were three 24 m² cabins for the different treatments: (CONTROL) without volatile emitters, (HD) with 1 0.01 mg/day emitter, and (LD) with 1 5 mg/day emitter. The relative humidity was 65% ± 10%, and the photoperiod was natural (approximately 14:10 h L: D) in all three cubicles. Environmental conditions were monitored and recorded using a Mithra clima data logger (ver. 1.01.03, Priva nutricontrol Ibérica S.L). In each of the three cubicles, 30 tomato plants were transplanted into individual 20-liter polyethylene pots filled with a mixture of sand and peat (1: 2 w:w, respectively). The pots were distributed in each cabin in four rows of five plants each (2 plants/m²). The typical cultivation techniques of greenhouse tomato cultivation in Spain were followed: staking to a guide for each plant, weekly pruning of secondary shoots, application of a standard nutrient solution for tomato using an automated irrigation system with an irrigation frequency adjusted to environmental conditions and an irrigation time of 15 min. The dispensers were placed 50 cm above the apex of the tomato plants and adapted in height according to the growth of the plants.

The dispensers were kept inside the corresponding cabins throughout the assay and samples were taken from the plants of each cabin 4 weeks after the implementation of treatment. From these samples, the transcriptional response of the defensive gene PIN2 (a marker gene for the jasmonic acid, JA, signaling pathway). Five repetitions per treatment were performed. The methodology followed for the extraction and quantification of gene expression was described by Pérez-Hedo et al. Journal of Pest Science 3: 543-554 (2015). The sequences of the primer used for the quantification of the PIN2 gene were forward primer: 5'-GAAAATCGTTAATTTATCCCAC-3' (SEQ ID NO: 9) and reverse primer: 5'-ACATACAAACTTTCCATCTTTA-3' (SEQ ID NO: 10), while for the constitutive gene EF1 the sequences were forward primer: 5'-GATTGGTGGTATTGGAACTGTC-3' (SEQ ID NO: 7) and reverse primer: 50-AGCTTCGTGGTGCATCTC-30 (SEQ ID NO: 8).

### Results

Expression studies showed that the lowest level of (Z)-3-hexenyl propionate emission (0.01 mg/day) did not affect exposed plants. However, a 5 mg/day emission significantly activated the jasmonic acid metabolic pathway (Figure 2).

### Example 3. Defensive activation assays in tomato via exposure under field conditions

### Methodology

To know the behavior of the dispensers loaded with (Z)-3-hexenyl propionate under real cultivation conditions, four commercial greenhouses for tomato cv Raf located in Xilxes (Castellón province), with a history of problems with *T. absolute* contamination at the end of winter, were selected. An experimental design of randomized blocks was carried out where each greenhouse was considered a block with two treatments per block and 4 repetitions per treatment. In these tomato greenhouses, the zoophytophagous predator *Nesidiocoris tenuis* Reuter (Hemiptera: Miridae) has been used, being released from the seedbed at the end of August and keeping phytophagous pest populations under control after being transplanted in the greenhouse at the end of August until the arrival of winter. However, the populations of this predator decline sharply under the environmental conditions that occur in winter. At the end of winter, their populations begin to recover slowly so that populations of *T. absolute* recover more quickly. It is common to resort to selective insecticides during the spring to keep *T. absolute* at bay. Therefore, these greenhouses were considered an ideal setting in which to confirm the effect on *T*. *absolute* found under laboratory conditions.

Cultivation began on 4 September 2018 with the transplant with a 0.4 × 1 m planting frame, which resulted in 1.2 plants per square meter (25,450 plants in 21,200 m²). Common cultivation techniques in the area were followed: the main stem was brought to two arms, secondary shoots and senescent leaves were pruned weekly, and a standard tomato nutrient solution was applied weekly through an automated drip irrigation system. The transplanted plants were inoculated in the seedbed with the predator *N. tenuis.* A dose of 1 *N. tenuis* per plant in the nursery was released, and *E. kuehniella* eggs were used as alternative prey (Urbaneja-Bernat et al. Journal of Applied Entomology 139: 61-167 (2015)). From the sowing date to the day the dispensers were hung, none of the greenhouses received any chemical treatment, and pest control resided only in the predator *N. tenuis.*

As mentioned earlier, 2 zones were selected in each of the greenhouses in which, on 22 February 2019, a dispenser (4-ml low-density (LD) polyethylene vial) loaded with 4 ml of (Z)-3-hexenyl was hung every 20 m² in one of them, and the other with the same surface area was used as a control treatment. In the four zones with volatile, a total of 260 dispensers were placed in a total surface area of 5,200 m² (equivalent to a density of 500 dispensers /ha). As described in Example 2, the dispensers' emission kinetics was studied using the gravimetric method.

Twenty plants randomly chosen by repetition were sampled weekly for 11 weeks, beginning 30 January 2019. First, the number of leaflets infested by *T. absolute* per plant was counted. Then the number of *N. tenuis* (adults and nymphs) throughout the apical third of each plant (leaves, flowers and shoots) was counted. Following the methodology described above, 6 samples were taken from the apical part of each plant per repetition, and these samples were immediately introduced into liquid nitrogen to quantify the expression of the PR1 and PIN2 genes (described in Example 1 and Example 2). The expression of these genes was quantified one day before, 4, and 8 weeks after hanging the dispensers in the greenhouses.

### Result

At the start of the assay, the expression of both marker genes for defense pathways was the same, but at 30 and 60 days, the expression of both genes was significantly higher in the plants exposed to the volatile compared to the plants in the control zone (Figure 3 A, B) (Table 1).

**Table 1.** Probability values for pairwise comparison of the transcriptional response of the defense genes PR1 (a marker gene for the SA signaling pathway) and PIN2 (a marker gene for the JA signaling pathway) in tomato plants exposed to (Z)-3-hexenyl propanoate [(Z)-3-HP] released in a 4-mL low-density (LD) polyethylene polymeric dispenser vial (Kartell) and in control tomato plants 24 hours before and 4 and 8 weeks after the establishment of dispensers in tomato greenhouses. Bold values correspond to statistically significant values. *t*-Test (P < 0.05).

| **Control *vs* (Z)-3-HP** | ***PR1*** | | ***PIN2*** | |
|---|---|---|---|---|
| | *t₁₄* | *P* | *t₁₄* | *P* |
| **1 day before** | 0.2464 | 0.8089 | 0.4412 | 0.6658 |
| **4 weeks** | **2.379** | **0.0321** | **3,037** | **0.0089** |
| **8 weeks** | **3.490** | **0.0040** | **2.263** | **0.0400** |

The population of the plant bug *N. tenuis* remained the same in both treatments (*F*₁, ₈₆ = 2.112; *P* = 0.150) (Figure 3C), but not so the infestation of *T. absolute,* which was significantly lower (about 58%) in the treatment with volatile (*F*_{1, 86} = 11.375; *P* < 0.0001) (Figure 3D). The volatile (Z)-3-hexenyl propanoate was emitted in a substantially consistent manner over the study period at a mean rate of 12.2 mg/day, according to the slope of the fitted model (Figure 3E), which represents an approximate application dose of 6 g/ha/day.

### Example 4. Defensive activation assays in citrus fruits via exposure under laboratory conditions

To demonstrate that exposure to the volatile (Z)-3-hexenyl propanoate activates defenses in citrus plants, seedlings of the Carrizo citrange rootstock (*Citrus sinensis* Osb. × *Poncirus trifoliata* L. Raf.). Two weeks after seed germination, the seedlings were individually transplanted into pots (8 × 8 × 8 cm). The plants were kept in a greenhouse with a plastic cover at approximate climatic conditions of 25 ± 5 °C, a relative humidity of 65% ± 5%, and a natural light photoperiod, about 14:10 h (light:dark). Carrizo citrange plants that had not received any pesticide treatment at 8 weeks of age (approximately 20 cm tall) were used. The volatile (Z)-3-hexenyl propanoate was obtained from Sigma-Aldrich (St. Louis, MO, USA). The volatile was exposed by using a 2 × 2 cm piece of filter paper on which 10 µl of a solution containing the corresponding volatile at a concentration of 1:10,000 [(Z)-3- hexenyl propanoate:methanol] was impregnated as an emitting source of the corresponding volatile (Pérez-Hedo, M. et al. Biocontrol, 63: 203-213 (2018)).

To activate a plant, two pieces of filter paper impregnated with the volatile were placed at the bottom of a 30 × 30 × 30 cm plastic cage (BugDorm-1 Insect Tents; MegaView Science Co., Ltd, Taichung, Taiwan) into which the plant was introduced. A control treatment was carried out in which the two pieces of filter paper were impregnated with 10,000 µl of methanol. Six repetitions per treatment were performed. The plants of both treatments were exposed to the volatile without disturbing for 24 hours in separate and isolated climatic chambers to avoid any interference from the volatile and were kept at 25 ± 2 °C, 65 ± 10% RH, and a 14:10 h (L: D) photoperiod. After 24 hours, the expression of 3 genes related to plant defensive activation was studied in both treatments: a precursor of JA jasmonic acid (LOX2) and two related to the downstream salicylic acid pathway (PAL) and a precursor (NPR1) were studied.

The methodology followed for the extraction and quantification of gene expression was described by Pérez-Hedo et al. Journal of Pest Science 3: 543-554 (2015). The primers used were forward primer: 5'- GAACCATATTGCCACTTTCG -3' (SEQ ID NO: 11) and reverse primer: 5'- CGTCATCAATGACTTGACCA -3' (SEQ ID NO: 12) for the quantification of LOX2, forward primer: 5'- CACATTCTTGGTAGCGCTTTG-3' (SEQ ID NO: 13) and reverse primer: 5'- AGCTACTTGGCTGACAGTATTC-3' (SEQ ID NO: 14) for PAL and forward primer: 5'- TACCTCCACCTCTCTCATTCTT-3' (SEQ ID NO: 15) and reverse primer: 5'- GTGCGAGAGAAGGTTAGCTATG-3' (SEQ ID NO: 16) for the NPR5 gene. The nucleotides of the constitutive gene glyceraldehyde 3-phosphate dehydrogenase (GAPDH) were forward primer: 5'-GGAAGGTCAAGATCGGAATCAA-3' (SEQ ID NO: 17) and reverse primer: 5'-CGTCCCTCTGCAAGATGACTCT-3' (SEQ ID NO: 18).

To assess the preference of *D. citri* females and the parasitoid *T. radiata* to Carrizo citrange plants that were previously exposed for 24 h to (Z)-3-HP in relation to intact plants, a Y-shaped olfactometer was used in the same way as that described in Example 1, and the plants were exposed to both volatiles as described above with the use of pieces of filter paper. A total of 40 valid replicates were recorded for each species for each pair of odor sources. Each individual was tested only once. Females that did not choose a side arm within 15 minutes were recorded as "no response" and excluded from data analysis. After recording five responses, the Y-tube was rinsed with soapy water, cleaned with acetone, and allowed to dry for 5 minutes. The odor sources were later switched between the left and right side arms to minimize any spatial effect on choice. The two types of plants (intact and induced) were used only once to assess the response of 10 females and were then replaced with new plants. The Y-tube experiment was performed under the following environmental conditions: 25 ± 1°C and 60 ± 10% RH.

The *D. citri* adults were obtained from a colony established at SWFREC (University of Florida), initially originating from citrus fruits located in their experimental plots. The parasitoids were collected in these experimental fields. Newly emerged adult females less than 5 days old were used in all the assays.

### Result

Exposure to intact Carrizo citrange plants' volatile (Z)-3-hexenyl propanoate significantly overexpressed the three markers studied (Figure 4A, B and C). Activated plants exposed 24 hours to (Z)-3-hexenyl propanoate turned out to be highly attractive to the parasitoid *Tamarixia radiata* (² = 6.914; *P* = 0.0043) and showed themselves to be indifferent to *Diaphorina citri* (² = 1.429; *P* = 0.1160) (*Figure 4D*).

### Example 5. Response to defensive activation in citrus fruits under field conditions

### Methodology

Efficacy of the use of dispensers loaded with HP in the field.

A Valencia orange field with a high presence of receptive shoots and a population of *D. citri* adults located in the experimental fields of SWFREC (University of Florida) was selected. The field had an approximate area of 0.3 ha. Sentinel plants that were hung inside the tree canopy were used. The sentinel plants were seedlings of *Murraya paniculata* with shoots receptive to the laying of *D. citri.* 7 seedlings with a dispenser (polymeric dispenser that was loaded with 1.5 ml of (Z)-3-hexenyl propanoate) and 7 seedlings without a dispenser were used. Each seedling was distributed in the field following a random block design (using a row of trees as a block), and one seedling with a dispenser and another one without was placed in each block. The seedlings were located at a minimum distance of 50 meters from each other to avoid any edge effect.

After 4 days of placing the seedlings, these were collected from the field and taken to the laboratory, where the number of eggs per shoot in each of the seedlings was counted under a binocular loupe.

### Result

### No differences were found between the number of receptive shoots between both treatments. As previously observed in the laboratory and in the greenhouse, the placement of the volatile dispensers managed to reduce D. citri by more than 70% (Figure 5).

Although specific experiments carried out with tomato and citrus plants have been described, the person skilled in the art will understand that the compound described in the present invention ((Z)-3-hexenyl propanoate) will be equally useful for its use in the protection of other types of plants by stimulating or inducing defense mechanisms. Likewise, although experiments in which tomato plants have been subjected to certain pests have been described, the person skilled in the art will understand that the compound described in the present invention will be equally useful for use in the protection of plants against other pests.

## Claims

1. Use of at least one compound of formula I 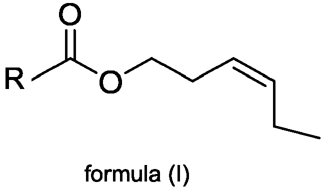 wherein R=-CH₃, -CH₂-CH₃, or -CH₂-CH₂-CH₃, or of a composition comprising same, for the protection of plants against pests by stimulating the natural defense mechanisms of said plants.

2. Use according to claim 1, wherein the compound of formula I is (Z)-3-hexenyl propanoate.

3. Use according to claim 1 or 2, wherein protection of the plants occurs through the repellency of pests and/or the attraction of parasitoids of the pests.

4. Use according to claim any of claims 1 to 3, wherein the defense mechanisms of plants cause the repellency of pests that are selected from the list comprising: whiteflies *Bemisia tabaci* (Gennadius) and *Trialeurodes vaporariorum* (Westwood), *Aleurothrixus floccosus* (Maskell), *Dialeurodes citri* (Ashmead) and *Paraleyrodes minei* laccarino (Hemiptera: Aleyrodidae), the thrips *Frankliniella occidentalis* Pergande, *Pezothrips kellyanus* Bagnall, *Chaetanaphothrips orchidii* (Moulton) and *Thrips tabaci* Lindeman (Thysanoptera: Thripidae), red spiders *Tetranychus urticae, Eutetranychus orientalis* (Klein), *Eutetranychus banksi* (McGregor), *T. urticae, Panonychus citri* (McGregor) Koch and *Tetranychus evansi* Baker & Pritchard (Acari: Tetranychidae), lepidoptera *Tuta absolute* (Meyrick), *Phyllocnistis citrella* Stainton (Lepidoptera: Gracillariidae), *Spodoptera exigua* Hübner and *Helicoverpa armigera* (Hübner) (Lepidoptera: Noctuidae) and psyllids *Diaphorina citri* Kuwayama (Hemiptera: Liviidae) and *Trioza erytreae* (Del Guercio) (Hemiptera: Psyllidae).

5. Use according to claim any of claims 1 to 4, wherein the defense mechanisms of the plants cause the attraction of the parasitoids selected from the list comprising species of the genera: *Encarsia* spp., *Aphytis* spp. *Cales* spp., *Eretmocerus* spp., *Aphelinus* spp., (Hymenoptera: Aphelinidae), *Aphidius* spp. *Lysiphlebus* spp. (Hymenoptera: Braconidae) *Metaphycus* spp., *Anagyrus* spp. (Hymenoptera: Encyrtidae), *Tamarixia* spp. *Citrostichus* spp., *Cirrospilus* spp., *Diglyphus* spp. (Hymenoptera: Eulophidae), *Trissolcus* spp., *Telenomus* spp. (Hymenoptera: Scelionidae) and *Trichogramma* spp. (Hymenoptera. Trichogrammatidae).

6. Use according to any of claims 1 to 5, wherein the protected plants are horticultural or citrus plants.

7. Use according to any of claims 1 to 6, wherein at least one compound of formula I, or of a composition comprising same, is applied to plants using dispensers.

8. Method for protecting plants against pests by stimulating the natural defense mechanisms of said plants, **characterized in that** it comprises contacting at least one compound of formula I, or of a composition comprising the same, with the plants: 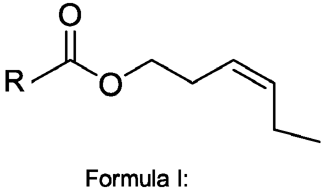 where R=-CH₃, -CH₂-CH₃ or -CH₂-CH₂-CH₃,

9. Method according to claim 8, **characterized in that** the compound of formula I is (Z)-3-hexenyl propanoate.

10. Method according to claims 8 or 9, **characterized in that** the compound of formula I, or of a composition comprising same, is contacted with the plant by means of dispensers.

11. Method according to claim 10, **characterized in that** the dispenser is a passive emitter consisting of a container of the compound of formula I configured to allow the diffusion of said compound into the environment surrounding the plant.

12. Method according to claim 10, **characterized in that** the dispenser consists of a nebulizer that produces an aerosol.

13. Method according to claim 10, **characterized in that** the dispenser is a liquid or gel composition that contains the compound of formula I and is formulated to allow diffusion of said compound into the environment.

14. Method according to any of claims 10 to 13, **characterized in that** the dose of compound applied to the environment surrounding the plants by the dispenser is between 25 mg/ha/day and 25 g/ha/day.

15. Method according to any of the preceding claims 8 to 14, **characterized in that** the natural defense mechanisms stimulated in plants cause the repellency of pests selected from the list comprising: whiteflies *Bemisia tabaci* (Gennadius) and *Trialeurodes vaporariorum* (Westwood), *Aleurothrixus floccosus* (Maskell), *Dialeurodes citri* (Ashmead) and *Paraleyrodes minei* laccarino (Hemiptera: Aleyrodidae), the thrips *Frankliniella occidentalis* Pergande, *Pezothrips kellyanus* Bagnall, *Chaetanaphothrips orchidii* (Moulton) and *Thrips tabaci* Lindeman (Thysanoptera: Thripidae), red spiders *Tetranychus urticae, Eutetranychus orientalis* (Klein), *Eutetranychus banksi* (McGregor), *T. urticae, Panonychus citri* (McGregor) Koch and *Tetranychus evansi* Baker & Pritchard (Acari: Tetranychidae), lepidoptera *Tuta absolute* (Meyrick), *Phyllocnistis citrella* Stainton (Lepidoptera: Gracillariidae), *Spodoptera exigua* Hübner and *Helicoverpa armigera* (Hübner) (Lepidoptera: Noctuidae); and psyllids *Diaphorina citri* Kuwayama (Hemiptera: Liviidae) and *Trioza erytreae* (Del Guercio) (Hemiptera: Psyllidae).

16. Method according to any of claims 8 to 15, **characterized in that** the natural defense mechanisms stimulated in plants cause the attraction of parasitoids selected from the list comprising species of the genera: *Encarsia* spp., *Aphytis* spp. *Cales* spp., *Eretmocerus* spp., *Aphelinus* spp., (Hymenoptera: Aphelinidae), *Aphidius* spp. *Lysiphlebus* spp. (Hymenoptera: Braconidae) *Metaphycus* spp., *Anagyrus* spp. (Hymenoptera: Encyrtidae), *Tamarixia* spp. *Citrostichus* spp., *Cirrospilus* spp., *Diglyphus* spp. (Hymenoptera: Eulophidae), *Trissolcus* spp., *Telenomus* spp. (Hymenoptera: Scelionidae) and *Trichogramma* spp. (Hymenoptera. Trichogrammatidae).

17. Method according to any of claims 8 to 16, **characterized in that** the plant is a horticultural plant or a citrus fruit.
